# EUROPEAN PATENT SPECIFICATION

(11) **EP 4 036 073 B1**
(45) Date of publication and mention of the grant of the patent: **18.10.2023**
(21) Application number: 20902107.0
(22) Date of filing: 12.08.2020
(51) Int. Cl.: C07C 5/333, C07C 11/06, C07C 11/09, B01J 8/02

(54) **PROCESS FOR THE DEHYDROGENATION OF LOWER ALKANES**
VERFAHREN ZUR DEHYDRIERUNG VON NIEDEREN ALKANEN
PROCÉDÉ DE DÉSHYDROGÉNATION D'ALCANES INFÉRIEURS

(30) Priority: 18.12.2019 CN 201911306207
(43) Date of publication of application: 03.08.2022
(73) Proprietor: Rezel Catalysts Corporation, Leshan, Sichuan 614000 (CN)
(72) Inventor: ZHUO, Runsheng, Leshan Sichuan 614000 (CN); FU, Yunzhi, Sichuan 614000 (CN)
(74) Representative: Bayramoglu et al.
(86) International application number: PCT/CN2020/108565
(87) International publication number: WO 2021/120644

(56) References cited:
- WO-A1-2015/152160
- CN-A- 102 019 178
- CN-A- 105 693 450
- CN-A- 106 029 612
- CN-A- 108 176 405
- CN-A- 110 903 155
- US-A- 2 419 997
- Instructional Materials Selection "Synthetic Rubber Production Process", Dalian University of Technology et al.: "One-Step Production of Butadiene from Butane by Dehydrogenation", Synthetic Rubber Production Process, 31 July 1964 (1964-07-31), pages 102-104, XP009528783,

## Description

### Technical field

The invention relates to a low-carbon alkane dehydrogenation process, more specifically, the invention relates to a process for realizing dehydrogenation reaction of low-carbon alkane with heat modulation which belongs to the technical field of petrochemical industry.

### Background

Low carbon olefin is the basic organic raw material with large demand and wide application in petrochemical industry. For example, propylene is an important basic chemical raw material, which is widely used in the production of chemical products such as polypropylene, isopropanol, isopropyl benzene, carbonyl alcohol, propylene oxide, acrylic acid, acrylonitrile, etc.; another important low carbon olefin butene is also widely used, such as mixed butene to produce high octane gasoline components, also to produce maleic anhydride, sec-butanol, n-heptene, polybutene, acetic anhydride and other products.

At present, China's demand for low-carbon olefin resources is still growing. Propylene supply mainly comes from the by-products of naphtha cracking to ethylene and heavy oil catalytic cracking process. Due to the growth of propylene demand, the supply of propylene is still insufficient in recent years. A large number of propylene products are still imported every year, and the original propylene source cannot fully meet the actual demand. The production processes of expanding propylene sources include propane dehydrogenation, olefin mutual conversion, olefin metathesis process, methanol to olefin and so forth. Among them, the process of propane dehydrogenation to propylene has attracted attention.

With the rapid growth of MTBE and alkylation unit, butene is also in short supply. Due to the large demand of dehydrogenated olefins for various chemical products, such as detergent, high octane gasoline, pharmaceuticals, plastics and synthetic rubber, dehydrogenation becomes an important preparation method. One application direction of this method is isobutane dehydrogenation to prepare isobutylene. Isobutylene can be polymerized to provide tackifier for adhesive, viscosity index additive for motor lubricating oil, impact resistance and oxidation resistance additive for plastics and components for oligomeric gasoline. Therefore, the method of isobutane dehydrogenation has also been paid attention.

China has relatively abundant light hydrocarbon resources such as liquefied petroleum gas and condensate, which contain a large amount of low-carbon alkanes, such as propane and butane. If the propane and butane can be effectively and directly converted to propylene and butene, the petroleum resources shall be fully used, which shall not only alleviate the problem of insufficient sources of low-carbon alkene, especially propylene and butene, but also simultaneously obtain high-value hydrogen. Therefore, it is necessary to develop a low-carbon alkane dehydrogenation process for industrial applications.

In order to meet the practical application requirements of the above-mentioned low-carbon olefins, R & D institutions around the world have developed a variety of low-carbon alkane dehydrogenation processes in the last century, including ABB Lummus Catofin process, UOP Oleflex process, Phillips' Star process and Linde PDH process * (please refer to Xiao Jintang's "C3-C4 olefin production process via alkane catalytic dehydrogenation (1 - 4)" [J]. Natural Gas Industry, 1994, 14 (2) - (4) and (6)).

Lummus' Catofin process is one of the main low-carbon alkane dehydrogenation processes, such as the one described in Graig RG, Delaney TJ, Buffalo JM. "Catalytic Dehydrogenation Performance of Catofin Process". Petrochemical Review. Houston. Dewitt. 1990*,* and the one in Feldman RJ, Lee E. "Commercial Performance of the Hourdry Catofin Process" 1992, NPRA*,* which are typical HOUDRY circulating fixed bed process (USP2419997). A cheap Cr₂O₃/Al₂O₃ catalyst was used, as described in USP6486370 and USP6756515 patents. The unit is comprised of several fixed bed reactors operating at a reaction temperature of about 600 °C. At high temperature and low pressure reaction conditions, propane absorbs a large amount of thermal energy through the catalyst bed to dehydrogenate and forms propylene, together with some side reactions. The catalyst needs to be regenerated every 15 minutes. This process has the advantages of high propane conversion, good propylene selectivity, strong raw material adaptability, and high on-line usage of the unit. Therefore, it has received more and more attention, especially in the application of isobutane dehydrogenation, and got widely employed.

As the dehydrogenation of hydrocarbons is a strong endothermic reaction, full heat utilization, heat balance and heat supplement are important factors for improving dehydrogenation conversion efficiency. For example, CN104072325A discloses a method to improve the dehydrogenation performance of low-carbon alkanes. In the dehydrogenation process, a fixed bed reactor with built-in electric heating tube is used to provide heat for the catalyst in the dehydrogenation process. The method not only reduces the temperature drop caused by the strong endothermic dehydrogenation reaction in the catalyst bed but also decreases the load of the electric heater for preheating the reactant gas, so as to reduce the thermal cracking of low-carbon alkanes in the preheating zone, which ultimately improves the performance of low-carbon alkanes dehydrogenation reaction and increases the yield of the target products.

The more common method of maintaining heat balance and heat reuse is to make full use of the heat generated during the regeneration of the catalyst. For example, CN105120997A disclosed a method of transferring the heat of exothermic catalyst regeneration reaction to an integrated fluidized bed reactor where the endothermic alkane dehydrogenation reaction takes at least a part of the regeneration heat. CN103003221A described a reaction in the presence of a mixture of inert heat exchange particles and catalyst particles. The heat exchange particles are heated in the heating zone and returned to the reaction zone to provide the required reaction heat. The catalyst is regenerated in a non-oxidizing atmosphere.

Undoubtedly, utilizing thermal coupling with exothermic reactions is a highly efficient way. CN101061084A completely hydrogenated all unsaturated hydrocarbons existing in the hydrocarbon stream before sending the stream to a dehydrogenation reactor and the heat released from the hydrogenation is substantially completely retained in the hydrocarbon stream, therefore, the energy needed for preheating the reaction stream to reach the reaction temperature is reduced. Also, coke is significantly reduced in the dehydrogenation reactor.

CN107223119A disclosed a method for converting paraffin, especially light paraffin such as C3-C8 paraffin into higher boiling liquid paraffin. The method coupled endothermic light paraffin dehydrogenation with exothermic alkene oligomerization for the heat needed.

Similar to the patent just mentioned, CN103772093A coupled alcohol dehydrogenation and low-carbon alkene hydrogenation in parallel tubing reactors. The heat released from the alkene hydrogenation reaction was used for the alcohol dehydrogenation reaction. The heat match between the endothermic and exothermic reactions eliminated the process heating and cooling, which simplifies the process, lowers the equipment investment and operating costs, reduces coking and extends the catalyst service life.

CN106365936A discloses a tubular hydrogen selective permeable membrane reactor, which allows alcohol liquid phase dehydrogenation to take place on one side of the membrane and hydrogen gas phase oxidation reaction on the other side of the membrane, that is, the hydrogen produced from the dehydrogenation reaction penetrates through the membrane which increases the reaction rate and improves the equilibrium conversion, and the hydrogen on the other side of membrane is oxidized in a controllable way to provide the heat for dehydrogenation, realizing the in-situ heating.

CN101165031A discloses a method for dehydrogenation of alkanes in a zoned reactor. In the exothermic reaction zone where oxygen and catalyst exist, a part of alkanes are exothermically converted into alkenes by oxidative dehydrogenation, and then the products from the exothermic reaction zone enter the endothermic reaction zone of the reactor where the remaining unconverted alkanes dehydrogenate with the help of carbon dioxide and other part of catalysts. Similar to this, CN106986736A also disclosed a similar zonal heat coupling method in the oxidative coupling reaction of methane.

Although the existing technology of dehydrogenation of alkanes to low-carbon alkenes has reported various improved processes and catalysts, involving the technology of heat generating agent, weak oxidant reaction and heat coupling, there are also new reports. For example, CN107074683A discloses a process method for catalytic dehydrogenation of alkanes to alkenes where Cr₂O₃ is used as catalyst and CO is introduced as the reducing gas during the reduction process to reduce the catalyst. The CO reduces the CuO component in the catalyst to form Cu and CO₂ and releases heat. The CO₂ generated reacts with the H₂ produced by dehydrogenation to form CO and H₂O.

USP2015/0259265A1 and CN106029612A disclosd a method of using heat-generating agent in the process of endothermic dehydrogenation of alkanes. In addition to commercial Catofin ^{®} 300 catalyst and inert α-alumina, heat generating materials (HGM) (loaded with copper, manganese and other metal elements on alumina) were also used in the process, which made the hydrocarbon react with multi-components in the catalyst bed, and air was used to regenerate the catalyst bed. The air and hydrocarbon used in the regeneration step increased the efficiency due to the low air / hydrocarbon ratio and near atmospheric pressure. USP7973207B2, USP7622623B, USP5108973, etc. also disclosed similar heat generating materials.

CN108176405A discloses dehydrating alkanes increased response auxiliary agent and its preparation method and application comprising 15m%~18m% of CaO, 70m%∼80m% of Al₂O₃, 6m%∼15m% of CuO and 0.01m%-3m% of oxide selected from groups VIII, VI, IA, IIA, and rare earth element or its mixture.

WO 2015/152160 A1 discloses a method for producing an unsaturated hydrocarbon by dehydrogenating a hydrocarbon using a dehydrogenation catalyst, it is stable over a long period of time by effectively suppressing the volatilization of zinc from the dehydrogenation catalyst. The dehydrogenation catalyst containing zinc as one of the active components to effect dehydrogenation of the hydrocarbon.

CN105693450A discloses processes and systems for producing olefins, including: dehydrogenating a first n-alkane to produce a first effluent; and dehydrogenating at least one of a first isoalkane or a second n-alkane to produce a second effluent. The first and second effluents may be compressed and fed to a common separation system to separate the effluents into two or more fractions. In some embodiments, each of the first and second dehydrogenation reaction zones may include two reactors, one reactor in each of the reaction zones operating in a dehydrogenation cycle, one operating in a regeneration cycle, and one operating in a purge or evacuation/reduction cycle.

Currently, due to the factors such as the pressure drop difference caused by catalyst uneven loading and the feed bias flow caused by improper process piping, it is difficult to achieve uniform temperature distribution and temperature dropping in the catalyst bed when the low-carbon alkanes dehydrogenate at the catalyst surface because of the reaction strong endothermic nature. The non-uniform temperature distribution and bias flow can seriously affect the catalyst life and the yield of low-carbon alkenes products. It is also not satisfactory in the aspects of the process severity, stability, operability, and operating cycle etc., and a constant improvement is needed.

### Summary

The catalytic dehydrogenation of propane, butane and other low-carbon alkanes is an endothermic reaction with the increase of molecular number. In the process of dehydrogenation of low-carbon alkanes, it is necessary to regenerate the catalyst frequently and provide the required heat at the same time. However, the high and uneven reaction and regeneration temperature of reactor bed, and the too strong cracking reaction of reaction system will lead to the decrease of selectivity of reaction; at the same time, it will also accelerate the carbon deposition rate of catalyst bed, so as to reduce or even inactivate the conversion performance of the whole reaction system. Therefore, it is the key factor to keep the catalyst bed temperature uniform during reaction and regeneration, and to reduce the reaction severity as much as possible.

The purpose of the invention is to overcome the shortcomings in the prior art, improve the temperature distribution of catalyst bed in fixed bed reactor, reduce the reaction and regeneration severity, improve the product yield, and provide an improved low-carbon alkane dehydrogenation process method.

Therefore, according to the above situation, the invention provides a method of low-carbon alkane dehydrogenation process, in particular, comprising:
(1) C₃-C₅ low-carbon alkane feed gas, CO and/or CO₂ process gas preheated to 200-500°C;
(2) The mixture gas entering the reactor and contacting with the Cr-Ce-Cl/Al₂O₃ dehydrogenation catalyst, Cu-Ce-Ca-Cl/Al₂O₃ thermal coupling additive, and heat storage/support inert alumina balls and converting under dehydrogenation condition 500-700°C, 10 to 100 kPa for 5 to 30 minutes, WHSV 0.1 to 5 h⁻¹;
(3) The low-carbon alkene and by-products produced entering the subsequent separation unit to separate out the low-carbon alkene, the hydrogen-rich gas, burning gas, and the unreacted low-carbon alkanes which is returned back to the reactor;
(4) The conversion process involving a periodic regeneration process of the catalyst bed which includes steam purging, heating with 560 to 730°C and 0.01 to 1 MPa hot air, evacuating and reducing catalyst. Each cycle takes 10 to 70 minutes; the aforementioned reduction process involving treating the catalysts bed with the hydrogen-rich gas separated out of the product stream or with commercially available hydrogen gas.

In the dehydrogenation process method of low-carbon alkane provided by the invention, the preferred reaction condition is that the preheating temperature of raw material and process gas is 300-450°C, the dehydrogenation is carried out under reaction temperature 540-650°C, reaction pressure 20-70 kPa, reaction time 10-20 min, and mass space velocity (WHSV) 0.3-2 h⁻¹.

In the dehydrogenation process method of low-carbon alkane provided by the invention, the preferred regeneration condition is to inject hot air of 600-700°C, 0.05-0.5 MPa during regeneration, and each cycle time is 20-35 minutes.

In the dehydrogenation process method of low-carbon alkane provided by the invention, the steam purging, evacuation and reduction process are conventional operations in the art, which are well known and daily used by ordinary technicians in the art.

The invention provides a dehydrogenation process method of low-carbon alkane, which is characterized in that in the single reaction regeneration cycle, the time ratio of dehydrogenation reaction, steam purging, heating catalyst bed, and evacuating / reduction is 1: (0.2-0.4): (0.8-1.1): (0.2-0.4); preferably 1: (0.25-0.35): (0.9-1.05): (0.25-0.35).

In the dehydrogenation process of low-carbon alkanes provided by the invention, the low-carbon alkanes refer to small molecular alkanes of C₂-C₅, also known as alkanes; preferably refer to low-carbon alkanes of C₃-C₄; more preferably, one or more of propane, isobutane and n-butane, which can be easily obtained by commercial purchase.

In the dehydrogenation process of low-carbon alkanes provided by the invention, the Cr-Ce-Cl/Al₂O₃ dehydrogenation catalyst contains 18-30 mol% of Cr₂O₃, 0.1-3 mol% of CeO₂, 0.1-1 mol% of Cl and 67-80 mol% Al₂O₃.

In the dehydrogenation process of low-carbon alkanes provided by the invention, the Cu-Ce-Ca-Cl/Al₂O₃ thermal coupling agent contains 5-30 mol% of CuO, 0.1-3 mol% of CeO₂, 10-35 mol% of CaO, 0.1-1 mol% of Cl, and 50-80 mol% of Al₂O₃.

In the dehydrogenation process of low-carbon alkanes provided by the invention, the ratio of the processing gas CO and/or CO₂ to the low-carbon alkane feedstock is 1-20 mol%; the preferred ratio is 1.5-5 mol%; The process is promoted by the reaction of the processing gas with the H₂ generated during dehydrogenation; the processing gas can be provided by the process flue gas which is separated out and returned to the reactor or obtained from commercially purchase.

In the dehydrogenation process of low-carbon alkanes provided by the invention, the volume ratio of the dehydrogenation catalyst, the thermal coupling agent, the heat storage inert alumina ball, and the supporting inert alumina ball is 1: (0.1 to 0.2): (0.4 to 0.7): (0.4 to 0.6); preferably is 1: (0.15 to 0.18): (0.5 to 0.6): (0.45 to 0.55); For the heat storage inert alumina balls and the supporting inert alumina balls used, their composition is Al₂O₃ ≥ 99.5 mol %, their heat capacity is 0.8368-1.4644 Joule/g °C (0.2-0.35 cal/g °C), and preferred heat capacity is 1.046-1.339Joule/g °C (0.25-0.32 cal/g °C); their maximum usage temperature is ≥ 1400°C, and they can be easily obtained through commercial purchase.

It is well known to those skilled in the art that the process method, unit and reaction system including catalyst and promoter constitute the content, system and features of the invention, and are different from the prior art, and are the most important factors affecting the catalytic conversion of hydrocarbons. Due to the great uncertainty in the mutual influence, it is difficult to obtain direct enlightenment from the prior art, and it is also difficult to obtain the expected results through simple permutation and combination experiments on the basis of the existing technology. It needs systematic research and exploration to get valuable results.

The process methodof alkane dehydrogenation reaction provided by the invention have high heat and reaction coupling conversion performance, which can make the temperature distribution of catalyst bed more uniform, so as to slow down the severe temperature difference of bed caused by factors such as bed pressure drop, feed bias, strong heat absorption, etc.

The invention reduces the inlet temperature or regeneration air flow of the regeneration air, thereby reducing the energy consumption of the unit; The decrease of the reactor inlet temperature, the reduction of the thermal cracking side reaction that may occur at the outlet of the heating furnace and inside the pipeline to the reactor bed, and the reduction of the heat loss all decrease the material consumption, and reduce the investment for the equipment.

Under the condition of keeping the total heat constant, the invention reduces the maximum temperature of the bed and the probability of deactivation of the catalyst at the top of the bed, moderates the temperature drop within a reaction cycle, and improves the selectivity while ensuring the constant conversion rate. Therefore, the invention simultaneously improves both the stability of the alkane dehydrogenation reaction process and the product yield of low-carbon alkene, which prolongs the service life of the catalyst. It is beneficial to the long-term operation of the dehydrogenation process.

### Brief Description of the Drawings

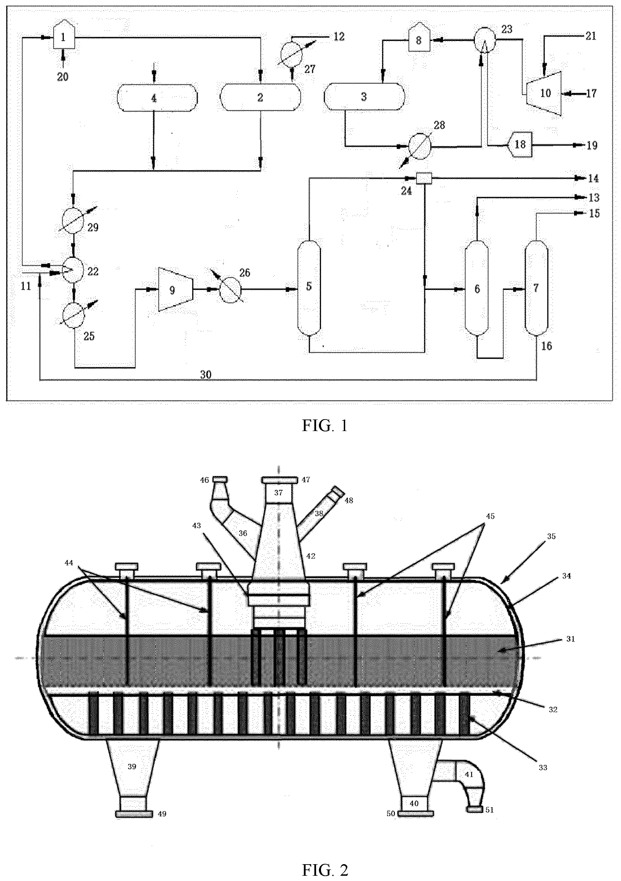

FIG. 1: The process flow diagram of low-carbon alkane dehydrogenation.
   where 1-feed heating furnace; 2-reactor under reaction; 3-regenerator under reaction; 4-reactor under purging; 5-flash separation tank; 6-de-ethane tower; 7-product separation tower; 8-air heating furnace; 9-product compressor; 10-gasifier; 11-raw low-carbon alkane; 12-process gas; 13, 20, 21-burning gas; 14-hydrogen-rich gas; 15-product low-carbon olefins; 16-recycle low-carbon alkanes; 17-air; 18-waste heat boilers; 19-exhaust gas; 22, 23, 27, 28, 29-heat exchangers; 24-coolers; 25, 26-condenser; 30-process pipe.)
FIG. 2: The structure diagram of fixed bed reactor for low-carbon alkane dehydrogenation where 31-catalyst bed; 32-catalyst bed refractory brick floor; 33-catalyst bed bottom refractory brick arch support; 34-fixed-bed reactor refractory lining; 35-reactor carbon steel shell; 36-low-carbon alkane feedstock inlet; 37-hot air inlet; 38-steam, process gas and reducing gas inlet; 39-hydrocarbon product outlet; 40-waste hot air outlet; 41-evacuation port; 42-air drying device; 43 -feed inlet deflectors; 44, 45-three-point thermocouples in the catalyst bed; 46, 47, 48, 49, 50, 51-programmed valves.

### Detailed Description of the Embodiments

With the help of the schematic diagrams it is clear that the invention provides an alkane dehydrogenation reaction process.

FIG. 1 is a flow chart of a low-carbon alkane dehydrogenation reaction process provided by the invention, and also a schematic diagram of a low-carbon alkane dehydrogenation reaction unit and reaction system.

FIG. 2 is the structure diagram of fixed bed reactor for low-carbon alkane dehydrogenation.

As shown in FIG. 1, the alkane dehydrogenation process unit includes: feed preheating furnace, air preheating furnace, and heating furnace which are connected to the reactor via process pipelines; 3-8 side-by-side fixed-bed reactors are controlled by program-controlled valves, which are used to keep the reactor rotation at different states such as reaction, regeneration and purging; an in-series separation unit connected to the reactor outlet is used for product separation; Compression and gasification equipment are used for the compression, circulation and gasification of air, product, process gas and burning gas respectively; Additionally, it also includes heat exchanger, condensation equipment and waste heat boiler connected via pipelines in the process for heat exchange, condensation and heat recovery of feed, products and recycle materials.

In the alkane dehydrogenation process of the invention, the reaction conversion process includes: low-carbon alkane feed gas 11, process gas 12 accounting for 1-20 mol% of low-carbon alkane feed gas, through process pipeline 30, preheating by heat exchanger 22, 27 and heating furnace 1 to 200-500°C, entering reactor 2 in reaction state from the top of the reactor; unconverted low-carbon alkane 16 and fresh feed gas 11 enter the reactor together; contact with chromium alumina dehydrogenation catalyst in fixed bed reactor 2, thermal coupling additive, inert heat storage alumina balls and supporting inert alumina ceramic balls.

Under the conditions of reaction temperature 500-700°C, reaction pressure 10-100 kPa, and mass space velocity (WHSV) 0.1-5h⁻¹, the reaction takes place and the reaction time is 5-30 min.

In the aforementioned single cycle, the time ratio of the dehydrogenation reaction, steam purging, catalyst bed heating and vacuum pumping/reduction is 1: (0.2-0.4): (0.8-1.1): (0.2-0.4).

The low-carbon olefins and by-products generated by the reaction are discharged from the lower part of the fixed bed reactor, heat-exchanged through heat exchanger 29 to generate steam, and further heat-exchanged with the heat exchanger 22 and the feed 11 and 16, then cooled with the condenser 25, and compressed with the product compressor 9 and further cooled and condensed with the condenser 26, and then entering into the subsequent separation unit 5, 6, 7 and 24 to separate out the low-carbon olefins 15, hydrogen rich gas 14 and side-products as burning gas gas 20 and 21. A part of the by-product burning gas 13, the unconverted low-carbon alkane 16, together with the fresh feed 11, passes through the full heat exchange (heat exchanger 22) and (heating furnace 1) heats up, and then circulates back to the reactor 2 at the reaction state for further conversion.

The conversion process includes the periodic regeneration process of the catalyst bed (31 in FIG. 2), which, through a set of program-controlled valves (46, 47, 48, 49, 50, 51 in FIG. 2), 3-8 fixed bed reactors are controlled to be at different states (reaction 2, purging 4 and regeneration 3); after the reaction conversion, the catalyst bed 31 stops feeding, and after the steam purging (reactor 4 at the purging state), 560-730°C and 0.01-1 MPa hot air enters the catalyst bed 31 for regeneration (reactor 3 at the regeneration state). The high temperature hot air is formed in the furnace 8 by air 17 and burning gas 21 after being gasified in the gasifier 10 and heat exchanged in the heat exchanger 23 before entering into the reactor 3 (reactor at the regeneration state). The high-temperature hot stream flows through the catalyst bed 31 in the reactor 3 at the regeneration state, then passes through the heat exchanger 28 to exchange the heat to generate steam, and after heat exchange in the heat exchanger 23 with cold air, the remaining heat is recovered via the waste heat boiler 18 and then vented 19.

After completing the regeneration process of the catalyst bed 31, the reactor goes to evacuation and reduction states, and then the dehydrogenation process is repeated; Each cycling time takes 10-70 minutes. The aforementioned reduction process includes a flash separation in the flash separation tower 5, and the hydrogen-rich gas 14 obtained in the cold box 24 (further separation if a PSA separation is employed) is used to reduce the catalyst bed 31 in the reactor 3 at the regeneration state. The catalyst bed 31 is packed with the dehydrogenation catalyst, the thermal coupling additive, the heat storage inert alumina balls and the supporting inert alumina ceramic balls having a volume ratio of 1: (0.1-0.2): (0.4-0.7): (0.4-0.6).

In the dehydrogenation process of low-carbon alkanes provided by the invention, the Cr-Ce-Cl/Al₂O₃ dehydrogenation catalyst in the catalyst bed 31 may be prepared following the steps and procedures given in the inventor's granted patent ZL200910210905.0, but the Cr-Ce-Cl/Al₂O₃ dehydrogenation catalyst of the invention has the composition of 18-30 mol% Cr₂O₃, 0.1-3mol% CeO₂, 0.1-1 mol% Cl and 67-80mol% Al₂O₃.

In the dehydrogenation process of low-carbon alkanes provided by the invention, the Cu-Ce-Ca-Cl/Al₂O₃ thermal coupling additive in the catalyst bed 31 can be prepared following the steps and procedures in the inventor's pending patents, application numbers 201711457256.5 and 201810119334.9; but the Cu-Ce-Ca-Cl/Al₂O₃ thermal coupling additive of the invention contains 5 -30 mol% of CuO, 0.1-3 mol% of CeO₂, 10-35 mol% of CaO and 50-80 mol% of Al₂O₃.

In the dehydrogenation process of low-carbon alkane provided by the invention, the heat storage inert alumina balls and the supporting inert alumina ceramic balls in the catalyst bed 31 have a composition of Al₂O₃ ≥ 99.5 mol %, a heat capacity of 0.8368-1.4644 Joule/g °C (0.2 to 0.35 cal/g °C), and a maximum use temperature of more than 1400°C, as an effective heat accumulator with guaranteed stability under harsh environments.

In the dehydrogenation process of low-carbon alkane provided by the invention, the reaction feed 11, 16 and the process gas 12 enter the reactor with carbon steel shell 35 and refractory lining 34 via the upper hydrocarbon inlet 36 and steam / gas inlet 38 of the reactor shown in FIG. 2, being guided by the feed inlet guide plate 43 and pass through from top to bottom the catalyst bed 31 supported by the arch support of refractory brick 33 and located on the refractory brick floor 32; The temperature change of catalyst bed is detected by the three-point thermocouple 44 and 45 in the bed; The reaction products are discharged from the lower part of the reactor via the hydrocarbon outlet 39, pass through the heat exchanger 29 and 22, cooler 25, compressor 9 and condenser 26, and then enter the subsequent separation equipment 5, 6, 7 and 24 for separation.

In the regeneration stage, after stopped the feed 11, 16 and 12, the steam enters the reactor (reactor 4) which is at the purging state from the inlet 38, purges from the top to the bottom the residual hydrocarbon in the catalyst bed 31 and discharges out from the outlet 39 at the bottom of the reactor; After that, the high-temperature hot air being dried by the air drying device 42 enters the reactor at the reducing state from the inlet 37 (Reactor 3) and passes through the catalyst bed 31 from top to bottom, which regenerates the catalyst and auxiliary agent and accumulates the heat in the catalyst, auxiliary agent, heat storage alumina and supporting alumina ceramic balls in the catalyst bed 31, also raises the bed temperature. The waste heat air is discharged from waste heat air outlet 40 at the lower part of the reactor. After exhausting the reactor through the evacuation port 41 at the lower part of the reactor, hydrogen rich gas 14 is introduced from the upper inlet 38 and goes through from top to bottom the catalyst bed 31 to reduce the catalyst and auxiliary agent. The exhaust gas is discharged through the exhaust port 41, and then the reactor enters the reaction state again. The state change and control of the reactor are achieved by a group of program-controlled valves, 46, 47, 48, 49, 50, 51.

The following embodiments are used to further describe the low-carbon alkane dehydrogenation process of the invention and its usefulness. As an illustrative explanation, the embodiments of the invention shall not be understood as restrictions to other generalized explanations of the invention given in the claims.

In the embodiment, the temperature change of the catalyst bed is monitored by three-point thermocouples in the bed; the analysis of the composition of the feed and reaction products is performed using an Agilent 6890N gas chromatography.

Other analytical methods can be found in relevant analytical methods (National Standard for Test Methods of Petroleum and Petroleum Products, China Standards Press, 1989) and (Analytical Methods of Petrochemical Industry (RIPP test methods), Science Press, 1990).

### Embodiment 1

Embodiment 1 illustrates the application usefulness of the low-carbon alkane dehydrogenation process, unit and reaction system in the propane dehydrogenation process.

Following the preparation procedures in the inventor's granted patent ZL200910210905.0, a 3 mm extrudate dehydrogenation catalyst with a composition of 23 mol% Cr₂O₃, 1 mol% CeO₂, 1 mol% Cl, and 75 mol% Al₂O₃ was prepared, and its surface area was 95 m²/g, bulk density was 1.05 g/ml, and crushing strength was 65 N/mm.

Following the steps in the pending patents of the inventors' 201711457256.5 and 201810119334.9, a 3 mm extrudate thermal coupling agent with a composition of 15 mol% CuO, 3 mol% CeO₂, 17 mol% CaO, and 65 mol% Al₂O₃ was prepared, and its surface area was 35m²/g , bulk density was 1.1g/ml, the crushing strength was 40 N/mm.

The test flow of the low-carbon alkane dehydrogenation reaction is shown in FIG. 1. The prepared 3 mm extrudate dehydrogenation catalyst, the prepared 3 mm extrudate thermal coupling agent, the 5mm heat storage Al₂O₃ ≥ 99.5 mol% with heat capacity of 1255 Joule/g oC (0.3 cal/g oC) and melting temperature ≥ 1700°C; the 8mm supporting inert alumina ceramic balls of Al₂O₃ >_ 99.5 m% with heat capacity 1255 Joule/g oC (0.3 cal/g oC) and usage temperature ≥ 1400°C are packed in the catalyst bed of eight industrial fixed bed reactors in a volume ratio of 1: 0.15: 0.5: 0.5, as shown in FIG. 2.

According to the process described in the invention, 8 fixed bed reactors are put into operation successively at 3 minute intervals. At any time, 3 reactors are in the dehydrogenation reaction process, 3 reactors are in the regeneration and reheating process, and 2 reactors are in the steam purging or evacuating / reduction process. The single cycle is about 25-30 minutes, including 10-15 minutes for dehydrogenation, 3 minutes for steam purging, 9 minutes for regeneration and reheating of catalyst bed, and 3 minutes for evacuating and reduction.

Table 1 shows the properties of industrial grade propane feedstock for propane dehydrogenation

**Table 1. Feedstock properties of propane dehydrogenation**

| Item | Composition/m% |
|---|---|
| Ethane | 1.2 |
| Propane | 95.4 |
| Propene | 2.5 |
| Diene & acetylene | 0.5 |
| C₄⁺ | 0.4 |

As the CO and CO₂ of the process gas, the industrial CO and CO₂ are those separated from the waste gas in the process unit of the invention.

Table 2 shows the operation conditions of dehydrogenation and regeneration when the low-carbon alkane dehydrogenation process method of the invention is applied to propane dehydrogenation

**Table 2. Operating conditions of propane dehydrogenation and regeneration**

| Item | Parameters |
|---|---|
| Feed temperature/°C | 590 |
| Pressure/kPa (absolutely) | 50 |
| Propane feed WHSV /hour⁻¹ | 0. 5 |
| Process gas WHSV /hour⁻¹ | 0.01 |
| Single pass reaction time/min | 10-15 |
| Temperature of regeneration air /°C | 670 |
| Pressure of regeneration air /kPa (absolutely) | 80 |

### Comparative Example 1

Referring to USP2419997, a commercially available Cr/Al₂O₃ industrial dehydrogenation catalyst and the similar industrial grade propane feed as in embodiment 1 were used, operating under the typical HOUDRY fixed bed dehydrogenation condition.

### Comparative Example 2

Referring to USP2419997, a commercially available Cr/Al₂O₃ industrial dehydrogenation catalyst, a similar industrial-grade propane feed as in Embodiment 1 and a commercially available Cu/Al₂O₃ heating generating material were used, operating under a typical HOUDRY circulating fixed bed dehydrogenation process condition.

### Embodiment 2

Embodiment 2 illustrates the comparison of the implementation results of the invention with Comparative Examples 1 and 2. Table 3 shows the comparison of the results of low-carbon alkane dehydrogenation process of the present invention, when applied to propane dehydrogenation reaction, with a typical HOUDRY circulating fixed bed dehydrogenation (Comparative Example 1), and those with a HOUDRY circulating fixed bed dehydrogenation with commercial heat generating material (Comparative Example 2). The catalyst life period not < 3 years is taken as the initial (SOR) and final (EOR) stages of operation.

**Table 3. Comparison of propane dehydrogenation of SOR - EOR catalyst life**

| Item | Embodiment 1 | Comparative Example 1 | Comparative Example 2 |
|---|---|---|---|
| Single pass propane conversion (SOR)/% | 50% | 44 | 45 |
| Single pass propane conversion (EOR)/% | 44% | 40 | 41 |
| Propene selectivity (SOR)/% | 86% | 84 | 84 |
| Propene selectivity (EOR)/% | 86% | 81 | 82 |

Compared with the operation of a typical HOUDRY circulating fixed bed dehydrogenation and the operation of a typical HOUDRY circulating fixed bed dehydrogenation with heat generating material, the invention has better propane single-pass conversion rate and propylene selectivity and achieves better propane dehydrogenation reaction efficiency.

### Embodiment 3

Embodiment 3 illustrates the process of low-carbon alkane dehydrogenation of the invention, and the implementation efficiency in the dehydrogenation process when it is applied to propane and isobutane mixed feed.

The dehydrogenation catalyst, thermal coupling agent, heat storage inert alumina balls and supporting inert alumina ceramic balls as prepared in Embodiment 1 are packed in the catalyst beds of the 8 industrial fixed bed reactors as shown in FIG. 2. The dehydrogenation of propane and isobutane mixed feed is carried out by following the process of the invention in Embodiment 1 and the process flow chart as shown in FIG. 1.

The data listed in Table 4 are the property data of propane and isobutane industrial mixed feed; The CO and CO₂ process gases were obtained in the same way as in Embodiment 1.

**Table 4. Property of propane and isobutane mixed feed**

| Item | Composition/m% |
|---|---|
| Ethane | 0.3 |
| Methyl acetylene | 0.02 |
| Propadiene | 0.02 |
| Propylene | 1.4 |
| Propane | 56.7 |
| Iso-butane | 37.2 |
| Iso-butene | 0.7 |
| n-Butane | 1.1 |
| n-Butene | 0.8 |
| 1,3-Butadiene | 0.2 |
| cis-Butene | 0.5 |
| trans-Butene | 1.1 |

Table 5 shows the operation conditions of dehydrogenation and regeneration when the low-carbon alkane dehydrogenation process method of the invention is applied to the dehydrogenation of propane and isobutane mixed feed.

**Table 5. Operating condition of dehydrogenation and regeneration of propane and isobutane mixed feed**

| Item | Parameters |
|---|---|
| Feed temperature/°C | 592 |
| Reactor pressure/kPa (absolutely) | 50 |
| Mixed feed WHSV /hour⁻¹ | 0.5 |
| Process gas WHSV /hour⁻¹ | 0.01 |
| Single pass reaction time/min | 10-15 |
| Regeneration air temperature/°C | 671 |
| Regeneration air pressure/ kPa (absolutely) | 80 |

### Comparative Example 3

Referring to USP2419997, dehydrogenation is carried out with a similar industrial grade propane and isobutane mixed feed as in Embodiment 3 and a commercially available Cr/Al₂O₃ industrial dehydrogenation catalyst under a typical HOUDRY fixed bed dehydrogenation process.

### Comparative Example 4

Referring to USP2419997, dehydrogenation is carried out with similar industrial grade propane and isobutane mixed feed as in Embodiment 1, a commercially available Cr/Al₂O₃ industrial dehydrogenation catalyst and a commercially available Cu/Al₂O₃ industrial heat generating material under a typical HOUDRY fixed bed dehydrogenation process.

### Embodiment 4

Example 4 illustrates the comparison of the implementation efficiency of the invention when it is applied to a mixed low-carbon alkane feedstock.

Table 6 shows the comparison of results of the HOUDRY circulating fixed bed dehydrogenation process (Comparative Example 3) with that having heat generating material (Comparative Example 4) when propane and isobutane mixed feed is used in the invention. The catalyst's lifetime is not less than 3 years as the initial and final operation period.

**Table 6. Comparison of dehydrogenation of propane and isobutane mixed feed at the beginning and end of catalyst life**

| Item | Example 3 | Comparative Example 3 | Comparative Example 4 |
|---|---|---|---|
| Single pass propane + isobutane mixed feed conversion (SOR)/% | 55% | 49 | 50 |
| Single pass propane + isobutane mixed conversion (EOR)/% | 45% | 41 | 42 |
| Propylene + isobutene selectivity (SOR)/% | 86% | 82 | 82 |
| Propene + isobutane selectivity (EOR)/% | 85% | 81 | 80 |

In comparison with the results of the typical HOUDRY circulating fixed bed dehydrogenation process and with those with heat generating material in dehydrogenation of a mixed propane and isobutane feed, the invention has better conversion and selectivity and obtains better implementation efficiency. The invention provides a low-carbon alkane dehydrogenation process which also has good implementation efficiency for the composition of more complex mixed low-carbon alkane feed and relatively more complex conversion process, and embodies good feed and process adaptability.

### Embodiment 5

Embodiment 5 illustrates the implementation efficiency of a low-carbon alkane dehydrogenation process of the invention when applied on reducing process severity, temperature difference, energy consumption, and material consumption.

In addition to the above implementation results obtained using industrial propane feed and mixed propane and isobutane feed, the comparison of operating condition data of dehydrogenation process in each embodiment also shows a good implementation efficiency.

The data listed in Table 7 is the comparison of the catalyst bed temperature and other operating conditions in the unit and the reaction system between the embodiment of the invention and the comparative example of the prior art, as well as the comparison of the process consumption data.

**Table 7. Comparison of operation conditions and process consumption between the embodiments of the invention and the prior art.**

| Item | Comparative example 1, 3 | Comparative example 2, 4 | Example 1, 3 |
|---|---|---|---|
| Temperature difference in catalyst bed | | | |
| Temperature difference between top and bottom of the bed/% | +8 | -4.2 | -5 |
| Temperature difference between middle and bottom of the bed /% | +4 | +3.2 | +3.5 |

| Comparison of severity | | | |
|---|---|---|---|
| Catalyst bed average maximum temperature /% | base | -2.5 | -3.4 |
| Reactor inlet temperature/% | base | -1.4 | -3.2 |
| Hot air inlet temperature/% | base | -1.7 | -4.8 |

| Comparison of process consumption | | | |
|---|---|---|---|
| Energy consumption/% | base | -3 | -8 |
| Mass consumption/% | base | -2 | -4 |

Compared with the prior art, the invention effectively reduces the temperature difference and the severity in the catalyst bed, and makes the temperature distribution more uniform; the invention also reduces the process energy consumption and material consumption to a certain extent, reflecting a better implementation efficiency.

These implementation results obtained under different operating conditions and severity are undoubtedly very beneficial to reduce the requirements on process unit and equipment, and on reaction system in terms of materials, design and operation.

## Claims

1. A process for dehydrogenating low-carbon alkanes, the process comprising:
(1) pre-heating C₃-C₅ low-carbon alkane feed gas, CO and/or COz process gas to 200-500°C;
(2) introducing the preheated mixture gas into a reactor and getting contact for 5-30 minutes with a Cr-Ce-Cl/Al₂O₃ dehydrogenation catalyst, a Cu-Ce-Ca-Cl/Al₂O₃ thermal generating agent, and heat storage/support inert alumina balls, and converting to products under the reaction conditions: temperature 500-700°C, pressure 10-100 kPa and WHSV 0.1-5 hour⁻¹;
(3) separating out the low-carbon alkenes and by-products in a separation unit and obtaining the low-carbon alkenes, hydrogen-rich gas and burning gas , and recycling the unreacted low-carbon alkane back to the reactor;
(4) periodically regenerating the catalyst bed via purging with steam, heating the catalyst bed with 560-730°C and 0.01-1 MPa hot air, evacuating the air, and reducing with Hz-rich gas stream, the cycle takes about 10-70 minutes.

2. The process of claim 1, wherein the dehydrogenation reaction and catalyst bed regeneration conditions are: pre-heating temperature of 300-450°C, dehydrogenation reaction temperature of 540-650°C, and pressure of 20-70 kPa, reaction time of 10-20 minutes, and WHSV of 0.3-2 hour⁻¹, and 600-700°C hot air and pressure 0.05-0.5 MPa in the process of regenerating , with the whole cycle taking 20 to 35 minutes.

3. The process of claim 1, wherein in a single reaction-regeneration cycle, the consumed time ratio of dehydrogenation : steam purge : hot air heating : vacuum/reduction is 1: (0.2-0.4) :(0.8-1.1) :(0.2-0.4).

4. The process of claim 1, wherein the low-carbon alkane is propane, isobutane or n-butane, or their mixture.

5. The process of claim 1, wherein the Cr-Ce-Cl/Al₂O₃ dehydrogenation catalyst contains 18-30 mol% Cr₂O₃, 0.1-3 mol% CeO₂, 0.1-1 mol% Cl, and 67-80 mol% Al₂O₃.

6. The process of claim 1, wherein the Cu-Ce-Ca-Cl/Al₂O₃ heat generating agent contains 5 to 30 mol% CuO, 0.1 to 3 mol% CeO₂, 10 to 35 mol% CaO, 0.1 to 1 mol% Cl and 50 to 80 mol% Al₂O₃.

7. The process of claim 1, wherein the proportion of the process gas CO and/or COz in the low-carbon alkane feed is 1-20 mol%.

8. The process of claim 1, wherein the filling volume ratio of the dehydrogenation catalyst, the heat generating agent, the heat storage inert alumina balls and the supporting balls is 1:( 0.1-0.2 ):( 0.4-0.7 ):( 0.4-0.6 ).

## Patentansprüche

1. Verfahren zum Dehydrieren von kohlenstoffarmen Alkanen, wobei das Verfahren umfasst:
(1) Vorwärmen von kohlenstoffarmem C₃-C₅-Alkan-Einsatzgas, CO und/oder CO₂-Prozessgas auf 200 bis 500 °C;
(2) Einleiten des vorgewärmten Gasgemischs in einen Reaktor und Inkontaktbringen für 5 bis 30 Minuten mit einem Cr-Ce-Cl/Al₂O₃-Dehydrierungskatalysator, einem Cu-Ce-Ca-Cl/Al₂O₃-Wärmeerzeugungsagens und Wärmespeicherungs-/Trägerkugeln aus inertem Aluminiumoxid und Umsetzen in Produkte unter den Reaktionsbedingungen: Temperatur 500 bis 700 °C, Druck 10 bis 100 kPa und WHSV 0,1 bis 5 h⁻¹;
(3) Abtrennen der kohlenstoffarmen Alkene und Nebenprodukte in einer Trenneinheit und Erhalten der kohlenstoffarmen Alkene, von wasserstoffreichem Gas und von Verbrennungsgas und Rückführen des nicht umgesetzten kohlenstoffarmen Alkans zurück in den Reaktor;
(4) periodisches Regenerieren des Katalysatorbetts durch Spülen mit Dampf, Erhitzen des Katalysatorbetts mit Heißluft mit 560 bis 730 °C und 0,01 bis 1 MPa, Evakuieren der Luft und Reduzieren mit H₂-reichem Gasstrom, wobei der Zyklus etwa 10 bis 70 Minuten dauert.

2. Verfahren nach Anspruch 1, wobei die Bedingungen der Dehydrierungsreaktion und der Katalysatorbettregeneration sind: Vorerwärmungstemperatur von 300 bis 450 °C, Dehydrierungsreaktionstemperatur von 540 bis 650 °C und Druck von 20 bis 70 kPa, Reaktionszeit von 10 bis 20 Minuten und WHSV von 0,3 bis 2 h⁻¹ und Heißluft mit 600 bis 700 °C und Druck 0,05-0,5 MPa bei dem Regenerationsprozess, wobei der gesamte Zyklus 20 bis 35 Minuten dauert.

3. Verfahren nach Anspruch 1, wobei in einem einzigen Reaktions-Regenerationszyklus das Verhältnis verbrauchter Zeit für Dehydrierung: Dampfspülung: Heißlufterwärmung: Evakuierung/Reduktion 1: (0,2 bis 0,4): (0,8 bis 1,1): (0,2 bis 0,4) beträgt.

4. Verfahren nach Anspruch 1, wobei das kohlenstoffarme Alkan Propanisobutan oder n-Butan oder ein Gemisch davon ist.

5. Verfahren nach Anspruch 1, wobei der Cr-Ce-Cl/Al₂O₃-Dehydrierungskatalysator 18 bis 30 Mol-% Cr₂O₃, 0,1 bis 3 Mol-% CeO₂, 0,1 bis 1 Mol-% Cl und 67 bis 80 Mol-% Al₂O₃ enthält.

6. Verfahren nach Anspruch 1, wobei das Cu-Ce-Ca-Cl/Al₂O₃-Wärmeerzeugungsagens 5 bis 30 Mol-% CuO, 0,1 bis 3 Mol-% CeO₂, 10 bis 35 Mol-% CaO, 0,1 bis 1 Mol-% Cl und 50 bis 80 Mol-% Al₂O₃ enthält.

7. Verfahren nach Anspruch 1, wobei der Anteil des Prozessgases CO und/oder CO₂ in dem kohlenstoffarmen Alkan-Einsatzgas 1 bis 20 Mol-% beträgt.

8. Verfahren nach Anspruch 1, wobei das Füllvolumenverhältnis des Dehydrierungskatalysators, des Wärmeerzeugungsagens, der Wärmespeicherungskugeln aus inertem Aluminiumoxid und der Trägerkugeln 1:(0,1 bis 0,2):(0,4 bis 0,7):(0,4 bis 0,6) beträgt.

## Revendications

1. Procédé de déshydrogénation d'alcanes à faible teneur en carbone, le procédé comprenant :
(1) le préchauffage du gaz d'alimentation d'alcane à faible teneur en carbone C₃-C₅, du gaz de traitement CO et/ou CO₂ à 200 à 500°C ;
(2) l'introduction du mélange gazeux préchauffé dans un réacteur et la mise en contact pendant 5 à 30 minutes avec un catalyseur de déshydrogénation Cr-Ce-Cl/Al₂O₃, un agent générateur thermique Cu-Ce-Ca-Cl/Al₂O₃ et des boules d'alumine inerte de stockage/support de chaleur, et la conversion en produits sous les conditions de réaction : température 500 à 700°C, pression 10-100 kPa et WHSV 0,1 à 5 heure⁻¹ ;
(3) la séparation des alcènes à faible teneur en carbone et des sous-produits dans une unité de séparation et l'obtention des alcènes à faible teneur en carbone, d'un gaz riche en hydrogène et d'un gaz de combustion, et le recyclage vers le réacteur de l'alcane à faible teneur en carbone n'ayant pas réagi ;
(4) la régénération périodique du lit de catalyseur par purge de vapeur, le chauffage du lit de catalyseur avec de l'air chaud à 560 à 730°C et à 0,01 à 1 MPa, l'évacuation de l'air et la réduction avec un flux de gaz riche en H₂, le cycle dure environ 10 à 70 minutes.

2. Procédé selon la revendication 1, dans lequel les conditions de réaction de déshydrogénation et de régénération du lit de catalyseur sont : une température de préchauffage de 300 à 450°C, une température de réaction de déshydrogénation de 540 à 650°C et une pression de 20 à 70 kPa, une durée de réaction de 10 à 20 minutes, et une WHSV de 0,3 à 2 heuré⁻¹, et de l'air chaud à 600 à 700°C et une pression de 0,05 à 0,5 MPa dans le processus de régénération, le cycle complet durant 20 à 35 minutes.

3. Procédé selon la revendication 1, dans lequel dans un cycle unique de réaction-régénération, le rapport de temps consommé de déshydrogénation : purge de vapeur : chauffage de l'air chaud : vide/réduction est de 1: (0,2 à 0,4): (0,8 à 1,1): (0,2 à 0,4).

4. Procédé selon la revendication 1, dans lequel l'alcane à faible teneur en carbone est du propane, de l'isobutane ou du n-butane, ou leur mélange.

5. Procédé selon la revendication 1, dans lequel le catalyseur de déshydrogénation Cr-Ce-Cl/Al₂O₃ contient 18 à 30 % en moles de Cr₂O₃, 0,1 à 3 % en moles de CeO₂, 0,1 à 1 % en moles de Cl et 67 à 80 % en moles d'Al₂O₃.

6. Procédé selon la revendication 1, dans lequel l'agent générateur de chaleur Cu-Ce-Ca-Cl/Al₂O₃ contient 5 à 30 % en moles de CuO, 0,1 à 3 % en moles de CeO₂, 10 à 35 % en moles de CaO, 0,1 à 1 % en moles de Cl et 50 à 80 % en moles d'Al₂O₃.

7. Procédé selon la revendication 1, dans lequel la proportion du gaz de traitement CO et/ou CO₂ dans la charge d'alcane à faible teneur en carbone est de 1 à 20 % en moles.

8. Procédé selon la revendication 1, dans lequel le rapport volumique de remplissage du catalyseur de déshydrogénation, de l'agent générateur de chaleur, des boules d'alumine inerte de stockage de chaleur et des boules de support est de 1:(0,1 à 0,2):(0,4 à 0,7):(0,4 à 0,6).
